# EUROPEAN PATENT APPLICATION

(11) **EP 4 049 698 A1**
(43) Date of publication of application: **31.08.2022**
(21) Application number: 20880129.0
(22) Date of filing: 09.11.2020
(51) Int. Cl.: A61M 5/178, A61M 5/31

(54) **DRUG DELIVERY DEVICE**

(30) Priority: 23.10.2019 RU 2019133647
(71) Applicant: Limited Liability Company "Next Bio", Saint-Petersburg, 191144 (RU)
(72) Inventor: RODIONOV, Petr Petrovich, d. Yukki, 188652 (RU); TARASENKO, Fedor Dmitrievich, Saint-Petersburg, 197022 (RU); ZHMAYLO, Michail Alexandrovich, Saint- Petersburg, 194358 (RU)
(74) Representative: Wilson Gunn
(86) International application number: PCT/RU2020/050317
(87) International publication number: WO 2021/080473

(57) **Abstract**

There is provided a drug delivery device, comprising: a housing; a drug reservoir installed in the housing; a rod movably installed in the housing so as to enable the drug to be discharged from the reservoir; an actuating mechanism capable of generating an actuating force; a dose-setting mechanism installed at least partially in the housing, wherein the dose-setting mechanism is capable of setting a drug dose and operatively coupled to the actuating mechanism; a tubular member provided with radial teeth, wherein the tubular member is operatively coupled in the housing to the dose-setting mechanism and to the rod such that the actuating force is transmitted to the rod in order to move the latter depending on the set drug dose; and a cylindrical member provided with at least one flexible element having a pawl, wherein the cylindrical member is locked in the housing such that the pawl is capable of jumping over at least one of the radial teeth during rotation of the tubular member.

## Description

### FIELD OF THE INVENTION

The present invention relates to medical equipment used for administering or injecting drugs, in particular to drug delivery devices.

### BACKGROUND OF THE INVENTION

Drug delivery devices are widely used in cases where a user needs to self-administer or self-inject a drug, in particular, to inject the drug regularly in set doses in accordance with an individual drug regimen.

Nowadays, drug delivery devices are commonly used among patients with diabetes mellitus since they allow such patients to manage their disease effectively on their own. The ease of use of such devices allows users without any special medical skills and/or any special medical education to use them; a procedure does not need to involve any skilled medical personnel.

The closest prior art to the present invention is a drug delivery device disclosed in RU 2696459 (IPC: A61M 5/00; publication date: 01 August 2019). It is to be noted that the drug delivery device according to RU 2696459 comprises a housing; a cylindrical driving mechanism; a ratchet mechanism comprising a gear provided with asymmetrical teeth having a sloping surface and a stop surface, wherein the gear is mounted on the cylindrical driving mechanism; a pawl, a spring which presses the pawl against the gear; and a cylindrical bearing element; wherein the pawl comprises three teeth and is installed so as to be radially movable in the cylindrical bearing element, wherein the cylindrical bearing element is installed inside the housing and rigidly connected thereto, and the gear is integrated with the cylindrical driving mechanism, wherein an assembly as obtained by mounting the pawl and the spring into the cylindrical bearing element and mounted on the driving mechanism and the driving mechanism are fixedly mounted on spacers in the housing; when the driving mechanism is rotated in a forward direction, the gear teeth and the pawl teeth slide along each other, and the pawl is lifted and lowered again under the action of the spring, thereby providing the reliable engagement and resistance to counter rotation of the driving mechanism and, therefore, the rod and simultaneously providing an accompanying sound during the forward rotation.

The drug delivery device according to RU 2696459 has a disadvantage. In particular, when such drug delivery device is used, especially for a long period of time, the action of the spring returning the pawl to its original position where it interacts with the gear teeth of the driving mechanism may weaken or become insufficient at some point to provide the return of the pawl, thereby resulting in that the accompanying sound is difficult to hear or not hearable at all. Therefore, a user may re-inject at least a part of a required drug dose, thereby administering an excessive drug dose, or may inject an incomplete drug dose, thereby preventing from administration of the required drug dose, so that it may lead to a patient having a false perception with regard to the injection/non-injection of the required drug dose.

Thus, developing an improved drug delivery device is an important concern in the art. In particular, the improved drug delivery device needs to prevent over-injection of a drug to a patient or non-injection of a required amount of the drug to the patient.

Consequently, a technical problem to be solved by the present invention is to develop a drug delivery device overcoming at least the aforementioned disadvantage of the known drug delivery device.

### SUMMARY OF THE INVENTION

It is an objective of the present invention to develop a drug delivery device solving at least the aforementioned technical problem.

To achieve the objective of the present invention, there is provided a drug delivery device, comprising: a housing; a drug reservoir installed in the housing; a rod movably installed in the housing so as to enable the drug to be discharged from the reservoir; an actuating mechanism capable of generating an actuating force; a dose-setting mechanism installed at least partially in the housing, wherein the dose-setting mechanism is capable of setting a drug dose and operatively coupled to the actuating mechanism; the device further comprises a tubular member provided with radial teeth, wherein the tubular member is operatively coupled in the housing to the dose-setting mechanism and to the rod such that the actuating force is transmitted to the rod in order to move the latter depending on the set drug dose; and a cylindrical member provided with at least one flexible element having a pawl, wherein the cylindrical member is locked in the housing such that the pawl is capable of jumping over at least one of the radial teeth during rotation of the tubular member.

The provided drug delivery device provides a technical effect which is improved reliability of the drug delivery device when a user using the drug delivery device needs to aurally control the injection process of a set drug dose, thereby allowing the user to use sound signals in the form of clicks or other sounds in order to focus his attention on the beginning, the course and the completion of the injection process of the set drug dose and to aurally control the stages of the injection process in a precise manner.

Furthermore, the provided drug delivery device provides a further technical effect which is expanded range of technical means intended to deliver drugs and utilizing various sound-generating means for notifying a user about the course of the injection process.

In another embodiment of the present invention, the cylindrical member is provided with three flexible elements, each comprising a pawl, and wherein the cylindrical member is locked such that the pawls of the flexible elements are arranged around the tubular member and offset with respect to each other at a predetermined offset angle, thereby allowing at least one of the pawls to alternatively jump over corresponding one of the radial teeth of the tubular member during rotation of the tubular member.

In a further embodiment of the present invention, each rotation of the tubular member by a predetermined angle corresponding to a unit of the drug dose allows only one of the pawls of the flexible elements to jump over corresponding one of the radial teeth of the tubular member. It further contributes to the aforementioned technical effect which is improved reliability of the provided drug delivery device when a user using the drug delivery device needs to aurally control the injection process of a set drug dose, in particular enables the user to aurally determine or identify the completion of delivery of one drug dose in a precise manner and, therefore, the total drug dose to be injected.

In yet another embodiment of the present invention, the predetermined rotation angle of the tubular member is 6 degrees, the radial teeth of the tubular member are offset by 18 degrees, and the pawls of the flexible elements are offset by 120 degrees.

The provided drug delivery device in accordance with any of the above embodiments of the present invention may be used to administer or inject a drug selected from a group consisting of insulins, insulin analogs, hormones, heparins, antihistamines, and their derivatives and analogs to a mammal (e.g., a human or animal).

### BRIEF DESCRIPTION OF THE DRAWINGS

FIG. 1 shows a fully assembled drug delivery device of the present invention with a cap fitted thereon.
FIG. 2 shows the drug delivery device of the present invention with the cap removed therefrom.
FIG. 3 is a general view of an outer housing used in the drug delivery device of the present invention shown in FIGS. 1-2.
FIG. 3a is a view of the outer housing shown in Fig. 3 as viewed from a distal end thereof.
FIG. 3b is a view of the outer housing shown in Fig. 3 as viewed from a proximal end thereof.
FIG. 3c is an enlarged view of the outer housing as viewed from the proximal end thereof, the enlarged view illustrating a partition in the outer housing.
FIG. 4 shows a general view of a holder for holding a cartridge as used in the drug delivery device of the present invention.
FIG. 5 shows a general view of the cartridge to be inserted into the holder shown in FIG. 4.
FIG. 6 shows a general view of a dose-setting drum with a push-button mounted thereon as used in the drug delivery device of the present invention.
FIG. 6a shows a view of the dose-setting drum with the push-button removed therefrom as viewed from a proximal end thereof.
FIG. 6b shows the push-button for the drum shown in FIG. 6a.
FIG. 7 shows a general view of the dose-setting drum with a connecting sleeve fitted thereon.
FIG. 7a shows a general view of the connecting sleeve used in the drug delivery device of the present invention.
FIG. 7b shows a view of the connecting sleeve shown in FIG. 7a as viewed from a distal end thereof.
FIG. 8 is a general view of the drug delivery device of the present invention with the following structural elements removed therefrom: the outer housing shown in FIGS. 3 and 3a-c, the connecting sleeve shown in FIGS. 7 and 7a-b, and the dose-setting drum shown in FIGS. 6 and 6a, in particular the general view illustrates a connecting tube which is engaged with an inner housing engaging with a bearing sleeve.
FIG 8a shows a part of the drug delivery device shown in FIG. 8 with the following structural elements further removed therefrom: the push-button shown in FIG. 6b, a ratchet wheel shown in FIG. 11, and the bearing sleeve shown in FIGS. 14 and 14a-b.
FIG. 8b shows a general view of the connecting tube used in the drug delivery device of the present invention.
FIG. 8c shows a side view of the connecting tube shown in FIG. 8a as viewed from a proximal end thereof.
FIG. 8d shows a side view of the connecting tube shown in FIG. 8a as viewed from a distal end thereof.
FIG. 9 shows a general view of a threaded screw installed within the connecting tube as used in the drug delivery device of the present invention.
FIG. 10 shows a general view of an annular element engaged on the threaded screw shown in FIG. 9 as used in the drug delivery device of the present invention.
FIG. 11 shows a general view of the ratchet wheel fitted on the connecting tube in the drug delivery device of the present invention.
FIG. 12 is a view illustrating the inner housing engaged with the bearing sleeve.
FIG. 12a is a view illustrating the part of the device shown in FIG 12 with the bearing sleeve removed therefrom.
FIG. 12b is a general view of the inner housing used in the drug delivery device of the present invention.
FIG. 12c shows a side view of the inner housing shown in Figure 12b as viewed from a distal end thereof.
FIG. 13 shows a rod with a disc-shaped element fitted thereon as used in the drug delivery device of the present invention.
FIG. 13a is a general view of the rod used in the drug delivery device of the present invention.
FIG. 14 is a general view illustrating the bearing sleeve engaged with a retaining ring as used in the drug delivery device of the present invention.
FIG. 14a is a view of the bearing sleeve as viewed from a proximal end thereof, wherein the view illustrates the bearing sleeve with flexible elements fitted thereon.
FIG. 14b is a view of the bearing sleeve as viewed from the proximal end thereof, wherein the view illustrates the bearing sleeve with flexible elements removed therefrom.
FIG. 15 is a general view of one of the flexible elements.
FIG. 16 is a general view of a blocking ring used in the drug delivery device of the present invention.

### DETAILED DESCRIPTION OF THE INVENTION

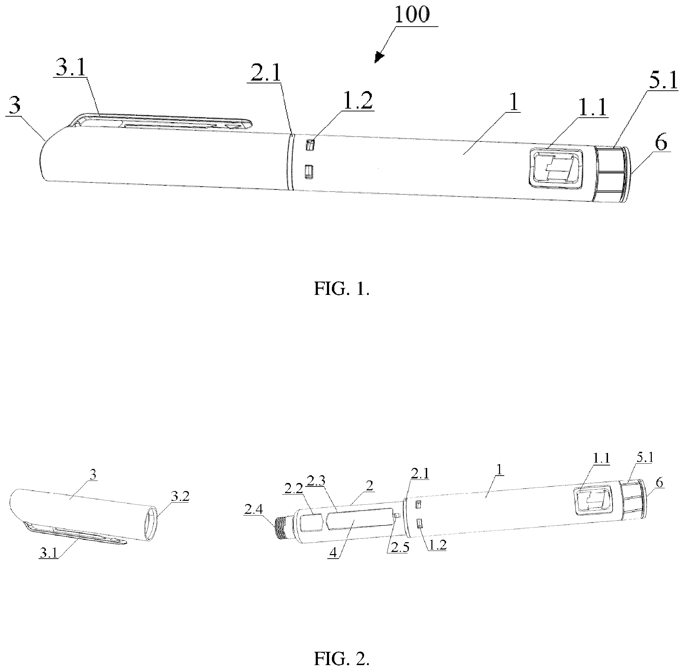

FIGS. 1 and 2 illustrate one embodiment of a drug delivery device 100 of the present invention as used to administer or inject a drug or medicine in a liquid form (e.g., insulin hormone when a patient is a person having type 1 or type 2 diabetes) to a patient and formed as an automatic syringe pen (hereinafter referred to as a syringe pen). It is to be noted that a user of the syringe pen 100 can be either the patient himself or any other person (e.g., a healthcare professional, a social worker, a relative of the patient, etc.) who may use the syringe pen 100 to administer or inject the drug to the patient.

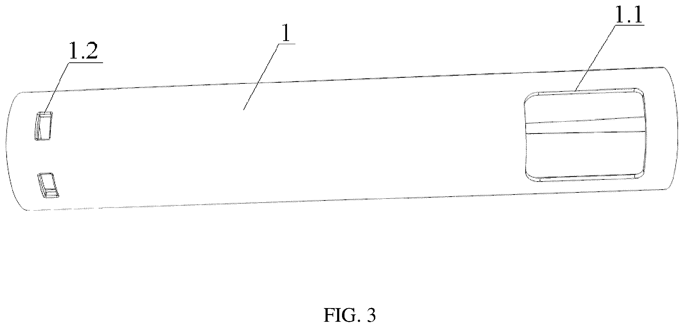

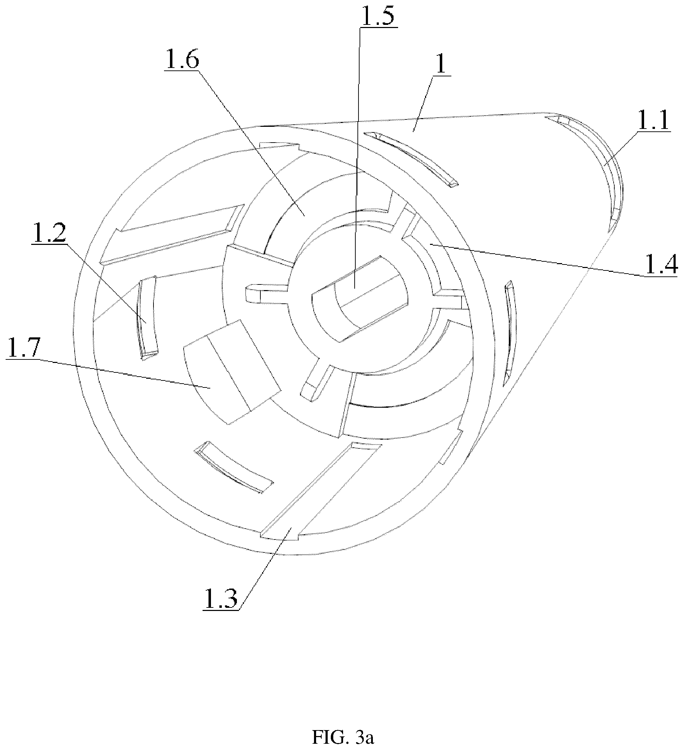

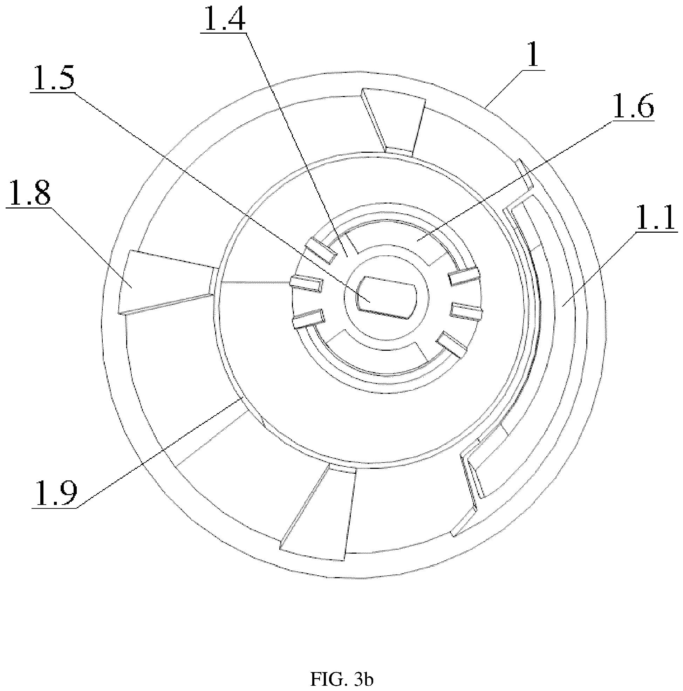

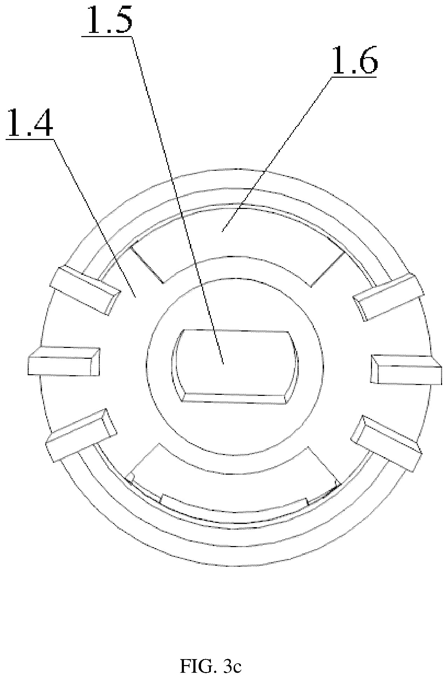

The syringe pen 100 comprises a tubular or cylindrical outer housing 1 illustrated in a general view in FIG. 3, wherein the outer housing 1 has a cylindrical inner cavity divided by a partition 1.4 into a first compartment of the outer housing and a second compartment of the outer housing, as shown in FIGS. 3a-c.

As shown in FIGS. 1-3 and 3a, the outer housing 1 is further provided at its distal end with fixing means, wherein the fixing means comprise diametrically opposite pairs of fixing slots 1.2 provided in a cylindrical wall of the outer housing 1 at a predetermined distance from the distal end of the outer housing 1 and from the partition 1.4. The fixing means further comprise diametrically opposite pairs of longitudinal grooves 1.3 provided on an inner surface of the outer housing 1 in its first compartment, wherein the longitudinal grooves 1.3 extend axially from the distal end of the outer housing 1 towards the partition 1.4. The fixing means also comprise two diametrically opposite blocking protrusions 1.7 capable of engaging with corresponding longitudinal cutouts 2.9 of a holder 2, as will be described below.

As shown in FIGS. 3a-c, the partition 1.4 in the outer housing 1 is formed as a disc-shaped or ring-shaped element provided with an axial central opening 1.5 and with two diametrically opposite arc-shaped cutouts 1.6, wherein the arc-shaped cutouts 1.6 are formed in the partition 1.4 such that they partially surround the central opening 1.5 thereof, and the central opening 1.5 of the partition 1.4 is shaped and sized to allow a piston rod 13 to pass therethrough, as will be described below.

As shown in FIGS. 3b and 3c, the outer housing 1 is further provided in its second compartment with an annular stop protrusion 1.9 provided on the inner surface of the outer housing 1 at a predetermined distance from its proximal end and from the partition 1.4, wherein the stop protrusion 1.9 is capable of being abutted by one of the ends of a connecting sleeve 7, as will be described in more detail below. As shown in FIG. 3b, the outer housing 1 is further provided in its second compartment with three fixing grooves 1.8 provided circumferentially on the inner surface of the outer housing 1 at a predetermined distance from each other and each extending substantially longitudinally from the proximal end of the outer housing 1 to the stop protrusion 1.9, wherein the fixing grooves 1.8 are capable of receiving the fixing ribs 7.1 of the sleeve, as will be described in more detail below.

As shown in FIGS. 3a and 3b, a viewing window 1.1 designed to visually control the set drug dose is provided at the proximal end of the outer housing 1, the proximal end of the outer housing 1 being opposite to the distal end thereof as used to insert the holder 2 thereinto.

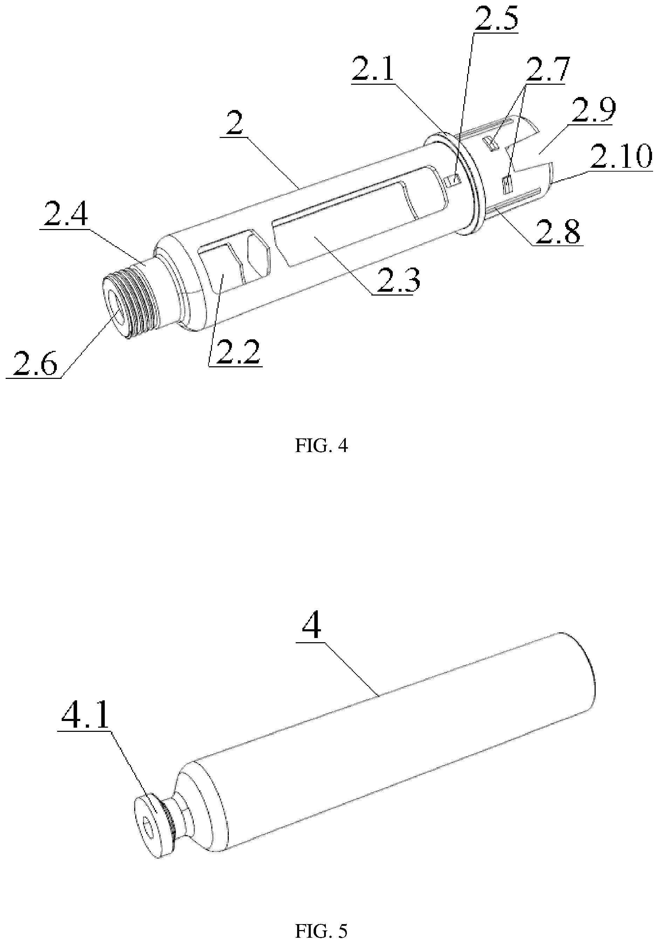

As shown in FIG. 4, the syringe pen 100 further comprises a tubular holder 2 for holding a drug reservoir 4 formed as an ampoule or a cartridge (see FIG. 5), wherein the reservoir 4 is divided externally by an annular flange 2.1 into a first portion of the holder 2, the first portion of the holder 2 being capable of being inserted into the first compartment of the outer housing 1, and a second portion of the holder 2, the second portion of the holder 2 being capable of being completely closed by a removable tubular cap 3 shown in FIGS. 1-2, wherein a diameter of the annular flange 2.1 is generally equal to an outer diameter of the outer housing 1 and to an outer diameter of the cap 3. It is to be noted that the outer housing 1 and the holder 2 are designed to be connected to each other to form a single protective casing or a single protective envelope, as will be described in more detail below.

In one embodiment, the syringe pen 100 may comprise a housing formed by a first housing portion functioning as the outer housing 1 as described below, and a second housing portion functioning as the holder 2 as described below.

As shown in FIG. 5, the cartridge 4 comprises a head 4.1 arranged on a distal end of the cartridge 4 and provided with a partition (not shown) sealing the cartridge 4 on the distal end thereof, wherein said partition is designed to be pierced to allow the drug contained in the cartridge 4 to be accessed. Further, the cartridge 4 is provided with a rubber plunger (not shown) sealing the cartridge 4 on a proximal end thereof, the proximal end of the cartridge 4 being opposite to the distal end of the cartridge 4, and allowing the drug to be discharged from the cartridge 4.

The holder 2 shown in FIG. 4 is provided with a head 2.4 on a distal end thereof, the distal end of the holder 2 being opposite to a proximal end thereof used to insert the holder 2 into the outer housing 1. A sleeve with a needle (not shown) can be fitted onto the cartridge head 4.1 such that one end of the needle is capable to pass through the central axial opening 2.6 provided in the holder head 2.4 and enter the cartridge 4 inserted in the holder 2, thereby allowing the needle to be in a fluid communication with the drug contained in the cartridge 4.

As shown in FIGS. 1 and 2, the cap 3 is provided externally with a clamp 3.1 for securing the syringe pen 100 to a garment.

Further, the holder 2 shown in FIG. 4 is provided on the outer surface thereof with two diametrically opposite holding protrusions 2.5, each being arranged between the flange 2.1 and a corresponding one of larger viewing windows 2.3; the cap 3 is provided with an annular recess 3.2 provided on an inner surface of the cap 3 on its proximal end used to fit the cap 3 onto the holder 2. It is to be noted that the cap 3 is used to protect the sleeve with the needle fitted onto the cartridge head 4.1 from external influences that could cause, e.g., sleeve damage and/or contamination, wherein taking the cap 3 off the holder 2 or removing it therefrom allows at least the following actions to be performed: fitting the sleeve with the needle onto the cartridge head 4.1, administering or injecting a set or predetermined drug dose to a patient and/or checking the residual amount of the drug in the cartridge 4, e.g., by using the below-described viewing windows 2.2, 2.3.

Further, the holder 2 shown in FIG. 4 is provided in its second portion with two diametrically opposite smaller viewing windows 2.2 and with two diametrically opposite larger viewing windows 2.3, said windows 2.2, 2.3 being arranged along a length of the holder 2 between the head 2.4 and the flange 2.1 and being aligned with respect to each other in an axial direction. When the cap 3 is removed from the second portion of the holder 2, the smaller and larger viewing windows 2.2, 2.3 allow various parts of the cartridge 4 inserted into the holder 2 to be visually accessed, thereby allowing the amount of drug remained in the cartridge 4 to be visually assessed.

The holder 2 shown in FIG. 4 is provided in its first portion with fixing means arranged on the outer side of the holder 2, wherein the fixing means comprise two diametrically opposite longitudinal cutouts 2.9 arranged such that they divide the proximal end of the holder 2 into substantially two diametrically opposite sectors 2.10, each being aligned in the axial direction with corresponding smaller viewing window 2.2 and larger viewing window 2.3. The fixing means of the holder 2 further comprise diametrically opposite pairs of longitudinal ribs 2.8 extending axially from the flanges 2.1 towards the proximal end of the holder 2. The fixing means of the holder 2 also comprise diametrically opposite pairs of latch protrusions 2.7 formed at a predetermined distance from the flange 2.1 and from the cutouts 2.9.

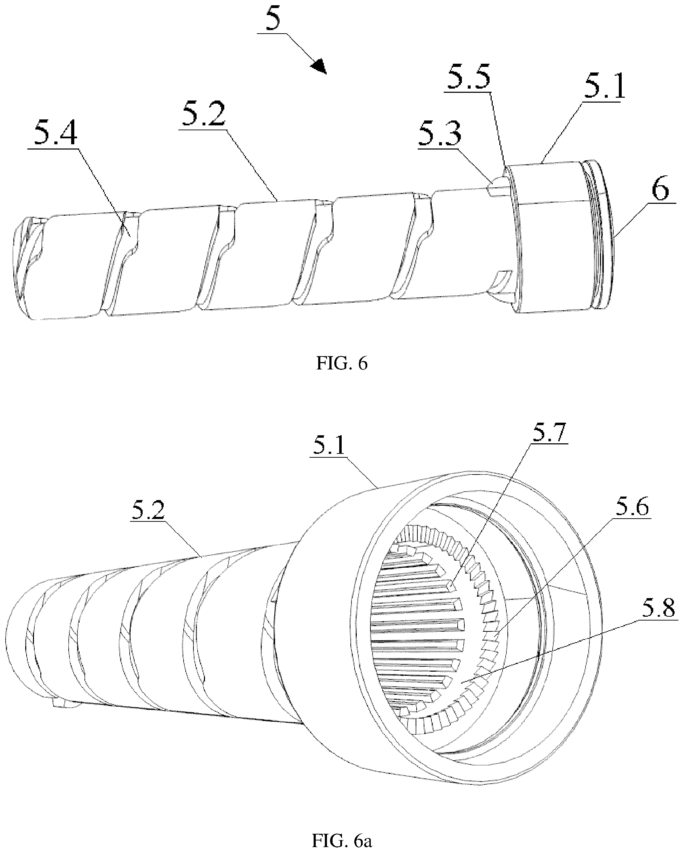

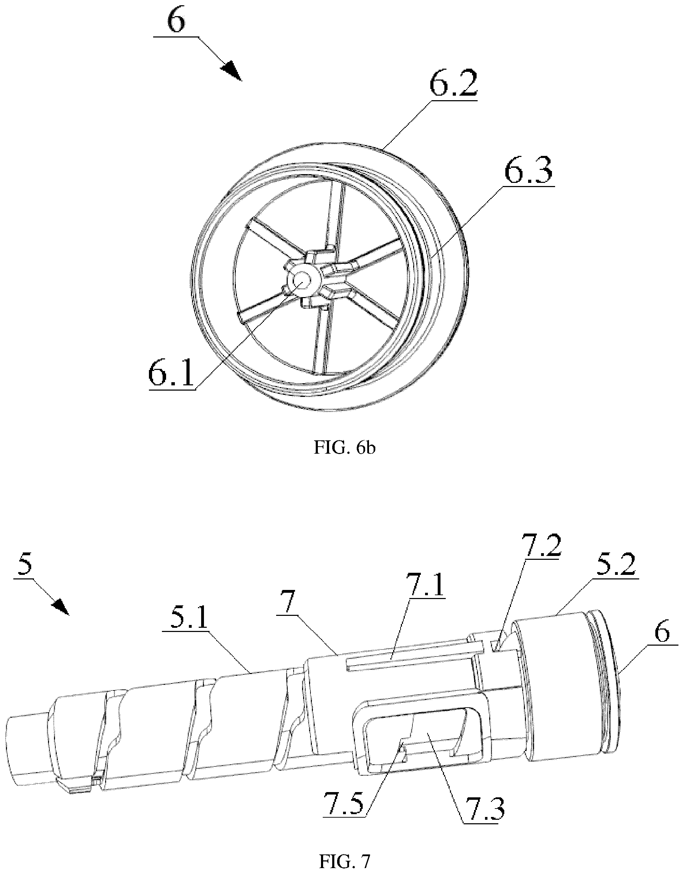

Further, the syringe pen 100 comprises a dosing mechanism comprising a rotatable dosing drum 5 (see FIGS. 6 and 6a). The drum 5 comprises a main portion 5.2 having a cylindrical inner cavity and a dose-setting head 5.1 provided on a proximal end of the drum 5, wherein the head 5.1 allows the dose to be incrementally set when a rotational force is applied thereto. The head 5.1 is integrally formed with the main portion 5.2 of the drum 5 and has a cylindrical inner cavity, the inner cavity of the head 5.1 being open to the inner cavity of the main portion 5.2. The head 5.1 is provided with an actuating mechanism formed as a push-button 6 (see FIG. 6b) installed on the proximal end of the drum 5 and designed to be pressed, in particular the push-button 6 may be pressed with a thumb to initiate the delivery of a set drug dose. Further, the head 5.1 is provided with a dose-setting grip (not shown) formed as axial ribs, wherein such axial ribs may be provided on an outer surface of the head 5.1 and circumferentially spaced around the head 5.1 in order to facilitate control over the head 5.1. As shown in FIG. 6b, the push-button 6 comprises a main portion 6.3, a head 6.2 and an axial pin 6.1, wherein the axial pin 6.1 is designed to be inserted into a seating 8.10 of the below-describbed connecting tube 8.

An outer diameter of the head 5.1 is substantially equal to the outer diameter of the outer housing 1 and exceeds an outer diameter of the main portion 5.2, so that a transition of a proximal end of the main portion 5.2 into a distal end of the head 5.1 forms externally an annular ledge 5.5 shown in FIG. 6. Furthermore, as shown in FIG. 6, the drum 5 is further provided with two diametrically opposite limiting protrusions 5.3 provided on an outer surface of the main portion 5.2, wherein each of the limiting protrusions 5.3 extends longitudinally from the annular ledge 5.5 towards a distal end of the drum 5.

As shown in FIG. 6, the main portion 5.2 has a helical groove 5.4 provided on the outer surface of the main portion 5.2 and extending substantially from the head 5.1 to the distal end of the drum 5, wherein the distal end of the drum 5 is opposite to a proximal end of the drum 5. Further, the main portion 5.2 is provided externally with a scale (not shown) formed by numerical labels indicative of a amount of drug dose units, in particular the drug dose units may be ranged from 0 to 60 with an increment of one unit, wherein the scale is spirally applied to the outer surface of the main portion 5.2 between turns of the helical groove 5.4.

As shown in FIG. 6a, the inner surface of the main portion 5.2 is further provided with ratchet teeth 5.7 formed as identical longitudinal ribs having a trapezoidal cross-section, wherein the ratchet teeth 5.7 are equally spaced around a circumference of the main portion 5.2 and extend axially substantially from the proximal end of the main portion 5.2 for a predetermined distance towards the distal end of the main portion 5.2, wherein a length of the ratchet teeth 5.7 corresponds to at least a portion of a length of the screw 10 or to an entire length of the screw 10. It is to be noted that each of the ratchet teeth 5.7 may correspond, e.g., to a dose increment by one dose unit or to accumulation of a single dose.

As shown in FIG. 6a, the head 5.1 is further provided with pawls 5.6 and an annular cavity 5.8, wherein the pawls 5.6 are formed as a rim of radial inclined teeth formed around a circumference of the inner surface of the head 5.1, and the annular cavity 5.8 is axially provided between the distal end of the head 5.1 and the pawls 5.6 such that the annular cavity 5.8 is substantially arranged between the pawls 5.6 and the ratchet teeth 5.7.

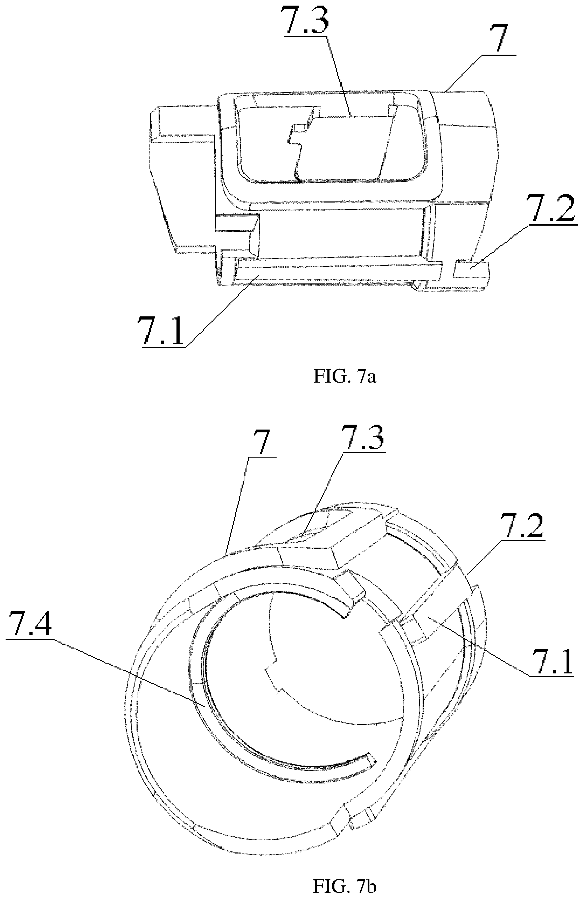

As shown in FIG. 7, the syringe pen 100 further comprises a connecting sleeve 7, wherein the connecting sleeve 7 has a cylindrical inner cavity and is provided with a viewing window 7.3, and dimensions of the viewing window 7.3 are smaller than those of the viewing window 1.1 of the outer housing, and the viewing window 7.3 is further provided with a dose indicator 7.5 capable of indicating a number of set drug dose units.

As shown in FIGS. 7 and 7a-b, the connecting sleeve 7 is further provided with three fixing ribs 7.1, wherein the fixing ribs 7.1 are spaced externally around a circumference of the connecting sleeve 7 and extend axially along at least a portion of a length of the connecting sleeve 7. The connecting sleeve 7 is further provided with two diametrically opposite limiting cutouts 7.2 provided on a proximal end thereof, the proximal end of the connecting sleeve 7 being used to fit the sleeve 7 onto the drum 5 when assembling the syringe pen 100, wherein the limiting cutouts 7.2 are capable of engaging with the above-described limiting drum protrusions 5.3. Further, the connecting sleeve 7 has a helical rib 7.4 provided on an inner surface of the connecting sleeve 7, wherein the helical rib 7 corresponds in shape and size to at least a part of a turn of the above-described helical groove 5.4.

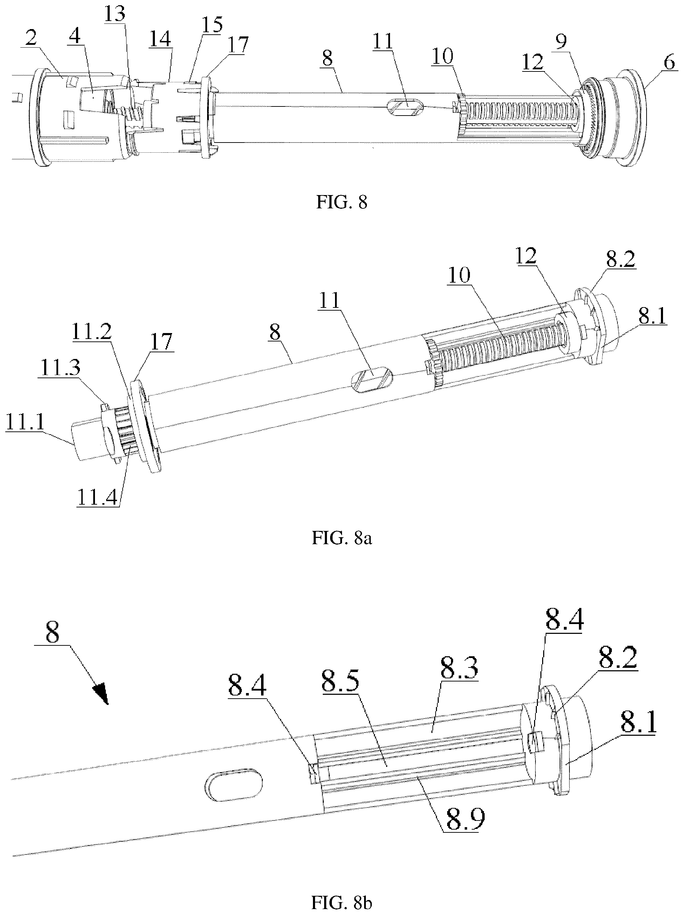

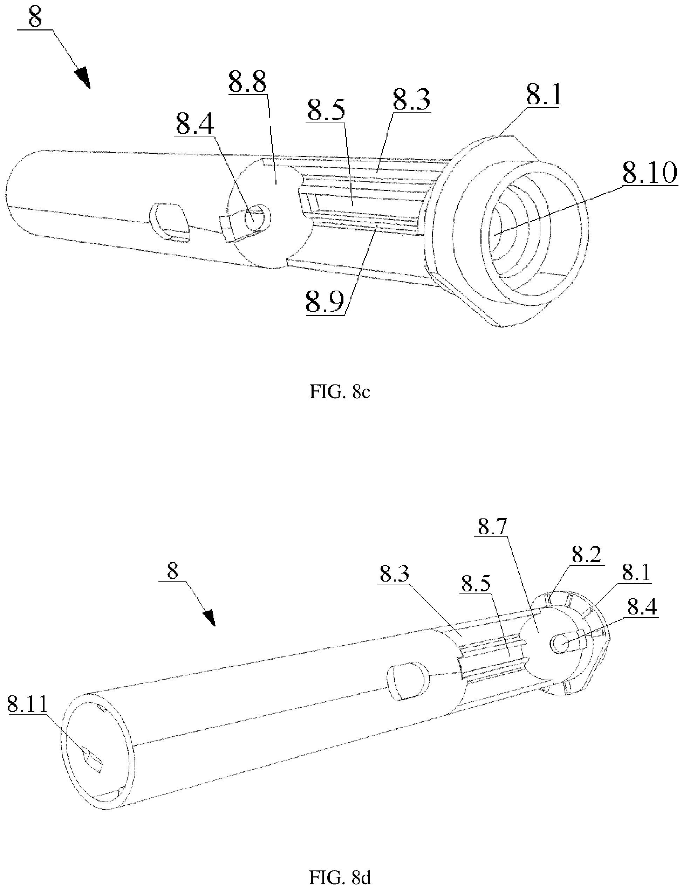

As shown in FIG. 8 and 8a-d, the syringe pen 100 further comprises a connecting tube 8 divided by solid partitions 8.7, 8.8 into a proximal compartment, a distal compartment and a central compartment. The proximal compartment is defined axially by the partition 8.7 and a proximal end of the connecting tube 8, wherein the proximal end of the connecting tube 8 is used to insert the connecting tube 8 into the drum 5. The distal compartment is defined axially by the partition 8.8 and a distal end of the connecting tube 8, wherein the distal end of the connecting tube 8 is opposite to a proximal end of the connecting tube 8. The central compartment is defined axially by the partition 8.7 and the partition 8.8 and partly exposed to form a cavity 8.3 having a generally cylindrical shape. The partitions 8.7, 8.8 on the side of the cylindrical cavity 8.3 are each provided with a mounting hole 8.4 having a generally circular shape, wherein the mounting holes 8.4 are arranged coaxially with each other. As shown in FIG. 8b, the cylindrical cavity 8.3 of the connecting tube 8 is further provided with a longitudinal inner protrusion 8.9, wherein the cylindrical cavity 8.3 is provided on the inner surface of the connecting tube 8 and extends axially from the partition 8.7 to the partition 8.8, and the inner protrusion 8.9 has a guide slot 8.5 extending axially substantially along an entire length of the inner protrusion 8.9.

As shown in FIG. 8c, the connecting tube 8 is provided at its proximal end with a seating socket 8.10 corresponding in shape and dimensions to an axial pin 6.1 of the push-button 6.

As shown in FIG. 8c, the connecting tube 8 is provided at its distal end with four substantially identical guide protrusions 8.11, wherein the guide protrusions 8.11 are spaced circumferentially on the inner surface of the connecting tube 8 and form two pairs of diametrically opposite guide protrusions, so that the guide protrusions in each of the pairs of guide protrusions are mirrored symmetrically with respect to each other. It is to be noted that the guide protrusions 8.11 are each curved and positioned in such manner that they substantially substantiallyform a section of a helical line and generally correspond to a turn of a thread formed by the helical groove 11.6 of the inner cylindrical housing 11, so that the guide protrusions 8.11 may be helically engaged with the helical groove 11.6 of the inner housing 11, thereby allowing the rotational or pivotal movement of the connecting tube 8 to be transmitted to the inner cylindrical housing 11, as will be described in more detail below.

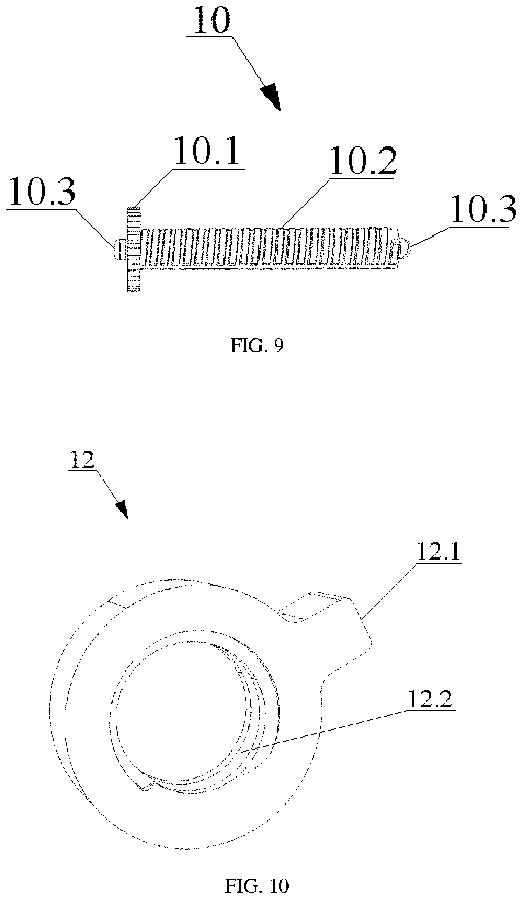

As shown in FIG. 9, the syringe pen 100 further comprises a screw 10 having a stem 10.2, the stem 10.2 being provided with a screw thread 10.2, and a toothed cap 10.1, wherein opposite ends of the screw 10 are provided with rounded mounting heads 10.3 arranged coaxially with each other.

Further, as shown in FIG. 10, the syringe pen 100 further comprises an annular member 12 provided on its inner surface with a helical rib 12.2 corresponding in shape and size to at least a portion of the helical thread 10.2 of the screw 10, wherein the annular member 12 is provided externally with a radial spike 12.1.

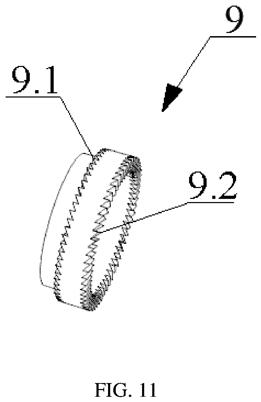

As shown in FIG. 11, the syringe pen 100 further comprises a ratchet wheel 9 provided with a rim 9.1 of inclined axial teeth and a rim 9.2 of inclined axial teeth, each of the rims 9.1, 9.2 being provided the outer surface of the ratchet wheel 9, wherein the inclined axial teeth in the rims 9.1, 9.2 are mirrored symmetrically with respect to each other.

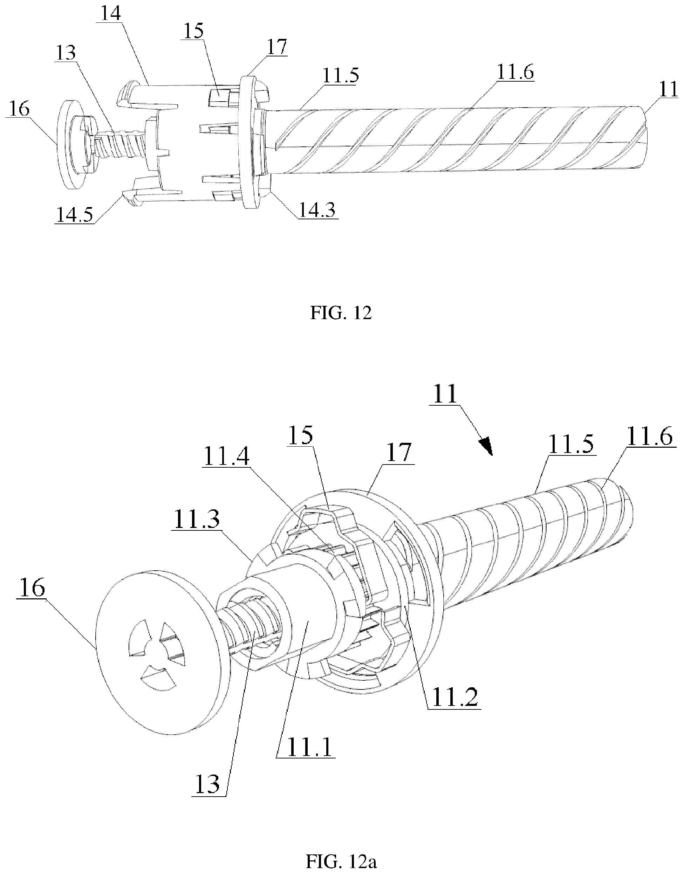

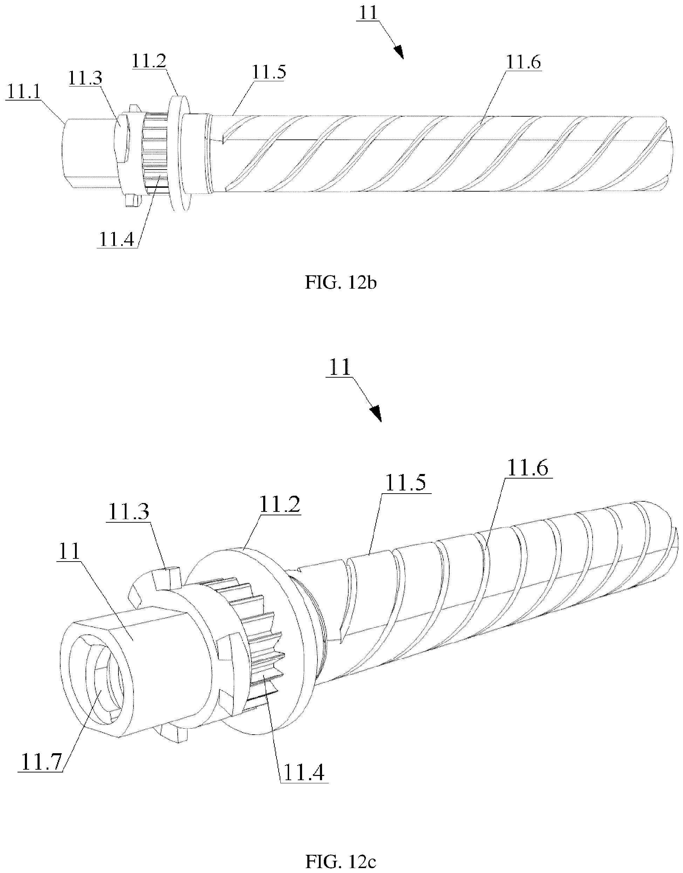

As seen in FIGS. 12 and 12a-c, the syringe pen 100 further comprises an inner cylindrical housing 11, wherein the inner housing 11 is formed as a hollow tube or tubular part and provided externally with an annular retaining shoulder 11.2. The annular retaining shoulder 11.2 is formed adjacent to a distal end of the inner housing 11, wherein the distal end of the inner housing 11 is opposite to a proximal end of the inner housing 11, the proximal end of the inner housing 11 being used to fit or put the connecting tube 8 thereon. The annular retaining shoulder 11.2 divides the inner housing 11 into an end portion 11.1 and a threaded portion 11.5, wherein the end portion 11.1 is defined axially by the retaining shoulder 11.2 and the distal end of the inner housing 11, and the threaded portion 11.5 is defined axially by the proximal end of the inner housing 11 and the retaining shoulder 11.2. The retaining shoulder 11.2 is further formed integrally with the threaded portion 11.5 and the end portion 11.1. The threaded portion 11.5 is provided externally with a helical groove 11.6 extending from the proximal end of the inner housing 11 substantially to the retaining flange 11.2, and the end portion 11.1 is provided on its inner surface with a screw thread 11.7, wherein the screw thread 11.7 is shaped and sized to allow the screw thread 11.7 to be helically engaged with the above-described guide protrusions 8.11 of the connecting tube 8.

As shown in FIGS. 12b-c, the end portion 11.1 is further provided with three arc-shaped protrusions or segments 11.3, wherein the arc-shaped segments 11.3 are formed externally in a center of the end portion 11.1 and spaced along a circumference of the end portion 11.1. The center of the end portion 11.1 is thickened, so that the end portion 11.1 has a larger outer diameter as compared to the rest of the end portion 11.1 of the inner housing 11, and the transition of the distal end of the end portion 11.1 to its thickened center forms an annular ledge on the outer side shown in FIGS. 12b-c. Further, the end portion 11.1 is provided externally with inclined radial teeth 11.4 equally spaced along the circumference of the end portion 11.1, in particular the radial teeth 11.4 are offset by 18 degrees, thereby forming a toothed rim or ring substantially arranged between the thickened center of the end portion 11.1 and the retaining flange 11.2.

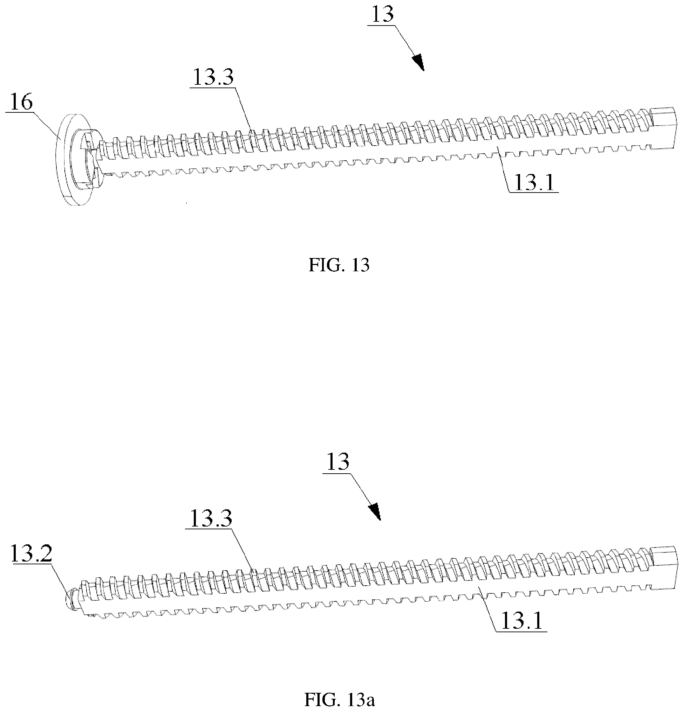

Further, as shown in FIGS. 13 and 13a, the syringe pen 100 comprises a piston rod 13 formed as an axial rod having a screw thread 13.3 provided on the outer side of the axial rod, wherein the screw thread 13.3 is arranged substantially over an entire length of the piston rod 13 and designed to engage with the screw thread 11.7 of the inner housing 11 to form a threaded connection. A portion of the screw thread 13.3 is cut or removed from two diametrically opposite sides of the piston rod 13 in the axial direction substantially along its entire length to form two diametrically opposite flat surfaces 13.1, so that the piston rod 13 is sized and shaped to pass through the central opening 1.5 provided in the partition 1.4 of the outer housing 1, thereby preventing or blocking the piston rod 13 from being turned.

Furthermore, as shown in FIG. 13a, a distal end of the piston rod 13 is provided with a seating head 13.2 configured to fit a disc-shaped element 16 thereon, wherein the disc-shaped element 16 is designed to engage with the plunger of the cartridge 4.

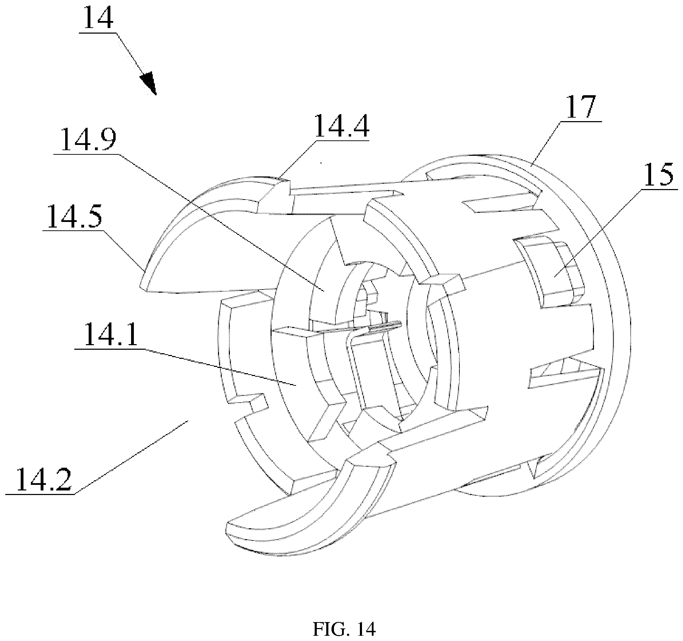

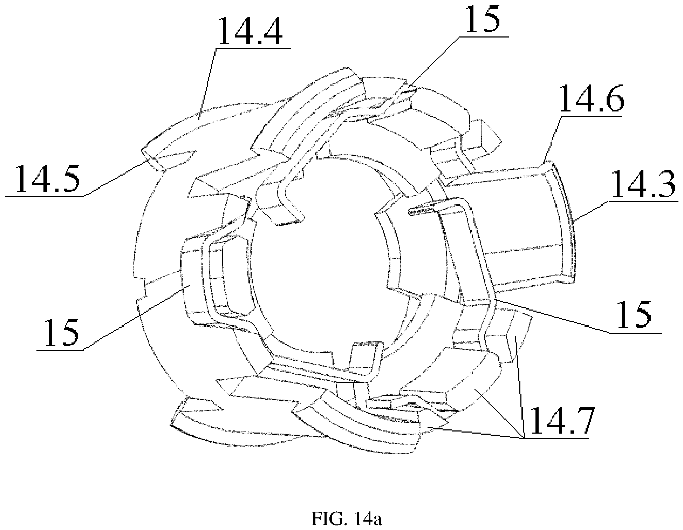

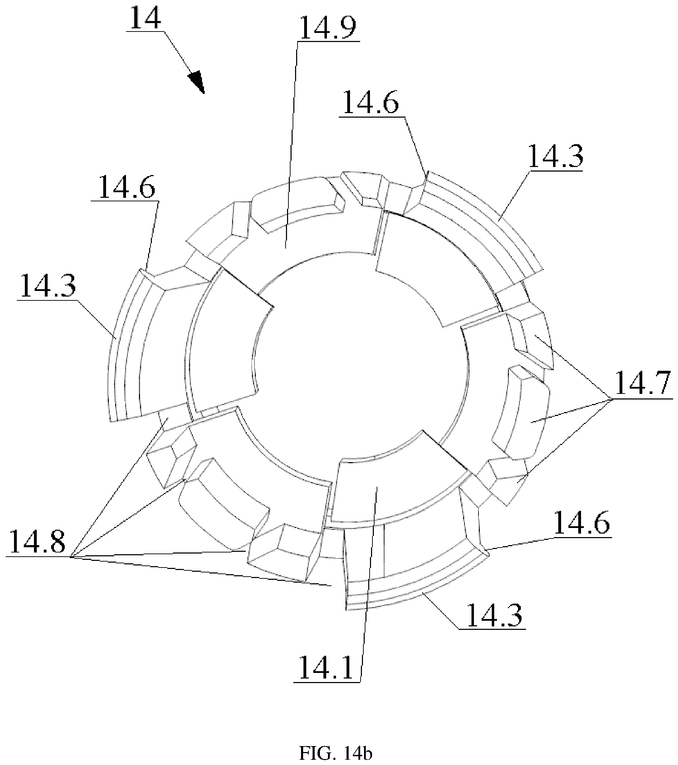

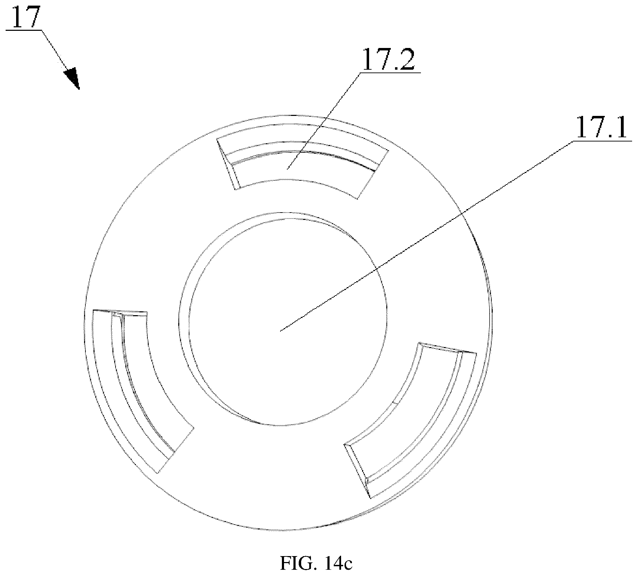

As shown in FIGS. 14 and 14a-b, the syringe pen 100 further comprises a bearing sleeve 14 formed as a cylindrical part having a substantially cylindrical inner cavity, wherein the bearing sleeve 14 is provided with three generally identical arc-shaped sectors 14.1 spaced circumferentially on an inner side of the bearing sleeve 14 so as to form three gaps or lumens of substantially identical size, each lumen being formed between corresponding two adjacent arc-shaped sectors 14.1. Further, the bearing sleeve 14 is provided with three further generally identical arc-shaped sectors 14.9 spaced circumferentially on the inner side of the bearing sleeve 14 so as to form three gaps or lumens of substantially identical size, each lumen being formed between corresponding two adjacent arc-shaped sectors 14.1. The arc-shaped sectors 14.9 are offset axially with respect to the arc-shaped sectors 14.1, so that they are arranged further away from the distal end of the bearing sleeve 14. The arc-shaped sectors 14.9 are formed and arranged circumferentially on the inner side of the bearing sleeve 14, so that they correspond in shape and size to the lumens formed by the arc-shaped sectors 14.1. The arc-shaped sectors 14.1 are formed and arranged circumferentially on the inner side of the bearing sleeve 14, so that they correspond in shape and size to the lumens formed by the arc-shaped sectors 14.9, and the arc-shaped sectors 14.1 in combination with the arc-shaped sectors 14.9 form a substantially closed circle or closed ring provided on the inner side of the bearing sleeve 14, as shown in FIG. 14b, and a generally annular space is formed between the arc-shaped sectors 14.1 and the arc-shaped sectors 14.9, wherein the space allows the above arc-shaped segments 11.3 of the inner housing 11 to be rotatably received therein. Thus, the annular space formed between the arc-shaped sectors 14.1 and the arc-shaped sectors 14.9 allows the arc-shaped segments 11.3 to freely rotate therein, wherein the rotation of the arc-shaped segments 11.3 is caused by the rotation of the inner housing 11 about its axis, and the arc-shaped sectors 14.1 and the arc-shaped sectors 14.9 block or prevent any axial displacement or movement of the arc-shaped segments 11.3 and, accordingly, any axial movement of the inner housing 11 during its rotation.

Furthermore, as shown in Fig. 14, the bearing sleeve 14 has two diametrically opposite axial cutouts 14.2 provided at the distal end of the bearing sleeve 14, wherein the distal end of the bearing sleeve 14 is opposite to the proximal end of the bearing sleeve 14, said proximal end being used to fit the bearing sleeve 14 onto the end portion 11.1 of the inner housing 11 to ensure that the end portion 11.1 is externally enclosed or surrounded. The axial cutouts 14.2 are formed such that they divide the distal end of the bearing sleeve 14 into substantially two diametrically opposite arc-shaped portions 14.5, wherein the arc-shaped portions 14.5 are shaped and sized to fit into corresponding arc-shaped cutouts 1.6 of the partition, and each of the arc-shaped portions 14.5 is provided with an arc-shaped hook 14.4 configured to engage with the partition 1.4 of the outer housing 1.

Further, as shown in FIGS. 14a-b, the bearing sleeve 14 further has axial cutouts 14.8 provided at the proximal end of the bearing sleeve 14, wherein the cutouts 14.8 form three separate groups of axial cutouts that are configured such that an elongated arc-shaped protrusion 14.3 is formed between each two adjacent groups of axial cutouts, and the axial cutouts 14.8 in each of these groups of axial cutouts form three shortened arc-shaped segments 14.7 arranged one behind the other around the circumference of the bearing sleeve 14 and separated from each other by corresponding axial cutouts from the group of axial cutouts 14.8. Thus, the bearing sleeve 14 has three elongated arc-shaped protrusions 14.3 provided at the proximal end of the bearing sleeve 14 and spaced along the circumference of the bearing sleeve 14, wherein three shortened arc-shaped segments 14.7 are formed between each pair of adjacent elongated arc-shaped protrusions 14.3, and a central shortened arc-shaped segment is separated from each of the two side shortened arc-shaped segments by a corresponding axial cutout from the group of axial cutouts 14.8. The elongated arc-shaped protrusions 14.3 are each provided with an arc-shaped hook 14.6, wherein the arc-shaped hooks 14.6 are configured to engage with the retaining ring 17.

As shown in FIG. 14c, the syringe pen 100 further comprises a retaining ring 17 having a central opening 17.1 shaped and sized so as to receive the threaded portion 11.5 of the inner housing 11 therethrough, wherein the retaining ring 17 is provided with three generally identical arc-shaped openings 17.2 formed around the central opening 17.1 and equally spaced around a circumference of the retaining ring 17, and the arc-shaped openings 17.2 are shaped and sized to receive corresponding elongated arc-shaped protrusions 14.3, thereby allowing the arc-shaped hooks 14.6 to engage with the retaining ring 17. It should also be noted that the retaining ring 17 is configured to be fitted onto the threaded portion 11.5 of the inner housing 11 from the proximal end thereof, so that the retaining ring 17 is seated on the threaded portion 11.5 of the inner housing 11 adjacent to the retaining flange 11.2 from the side of the threaded portion 11.5 of the inner housing 11. Thus, when the elongated arc-shaped projections 14.3 of the bearing sleeve 14 are inserted into corresponding arc-shaped openings 17.2 of the retaining ring 17 to allow the arc-shaped hooks 14.6 of the bearing sleeve 14 to engage with the retaining ring 17, and when the arc-shaped portions 14.5 of the bearing sleeve 14 are inserted into corresponding arc-shaped cutouts 1.6 of the partition of the outer housing 1 to allow the arc-shaped hooks 14.4 to engage with the partition 1.4, one side of the bearing sleeve 14 is rigidly and releasably attached to the retaining ring 17 so as to have the retaining ring 17 fitting tightly to the retaining flange 11.2, and the other side of the bearing sleeve 14 is rigidly and releasably attached to the outer housing 1, thereby allowing the bearing sleeve 14 to be retained in a required position with respect to the end portion 11.1 of the inner housing 11, in particular with respect to the arc-shaped segments 11.3 and the radial teeth 11.4 of the inner housing 11, and, accordingly, preventing the bearing sleeve 14 and the retaining ring 17 from being axially moved and being rotated or pivoted with respect to the inner housing 11; however, the inner housing 11, in particular the end portion 11.1 and the threaded portion 11.5 thereof, remains rotatable with respect to the bearing sleeve 14 and the retaining ring 17 attached thereto.

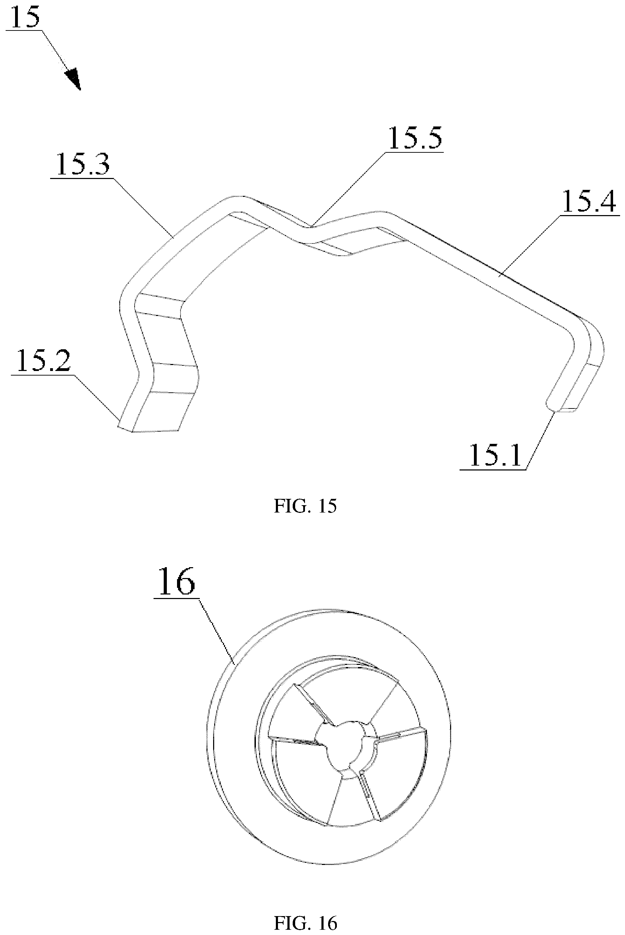

As shown in FIG. 14a, the bearing sleeve 14 is also provided with three generally identical spring or flexible elements 15, each being formed as a curvilinear or curved elongated plate element shown in FIG. 15 or as a leaf spring. As shown in FIG. 15, each flexible element 15 is bent so that it has a free end 15.2, a trapezoidal fixing portion 15.3 and a free elongated portion 15.4 provided with a hook or pawl 15.1, wherein the trapezoidal fixing portion 15.3 is connected to the free elongate portion 15.4 via a bent transition 15.5. As shown in FIG. 14a, each flexible element 15 extends through two central axial cutouts of the axial cutouts 14.8 of the bearing sleeve 14, wherein the cutouts separate the central shortened arc-shaped segment of the shortened arc-shaped segments 14.7 from the two side shortened arc-shaped segments of the shortened arc-shaped segments 14.7 such that the trapezoidal fixing portion 15.3 thereof encloses the central shortened arc-shaped segment on the outer side of the bearing sleeve 14 and at least partially abuts the central shortened arc-shaped segment, and the free end 15.2 and the bent transition 15.5 each enclose a corresponding one of the side shortened arc-shaped segments on the inner side of the bearing sleeve 14 and at least partially abut the enclosed shortened arc-shaped segment, thereby allowing each flexible element 15 to be rigidly fastened on the bearing sleeve 14, wherein the free elongated portion 15.4 of each flexible element 15 with the pawl 15.1 faces the cylindrical inner cavity of the bearing sleeve 14. Furthermore, it is to be noted that the above connection between the flexible elements 15 and the bearing sleeve 14 allows the pawls 15.1 to be oriented in a desired manner in the inner cavity of the bearing sleeve 14, so that the tips of the pawls 15.1 each can interact with or can be engaged with a corresponding one of the radial teeth 11.4 of the inner housing 11, wherein the pawls 15.1 of the flexible elements 15 locked to the bearing sleeve 14 are generally circumferentially and equally spaced from one another, in particular, the tips of the pawls 15.1 are circumferentially angularly shifted or offset by 120 degrees with respect to one another.

Thus, in a delivery mode for delivering a set drug dose to an injection site of a patient, when the inner housing 11 is rotated with respect to the bearing sleeve 14, the bearing sleeve 14 along with the flexible elements 15 secured thereto having a locked position with respect to the end portion 11.1 of the inner housing, at least one of the pawls 15.1 is allowed to jump over a corresponding one of the radial teeth 11.4 of the inner housing 11, thereby generating or producing sounds in the form of clicks or other sharp sounds audible to the user. In particular, in delivery mode for delivering a set drug dose to the injection site of a patient, rotation of the inner housing 11 by 6 degrees causes only one of the three pawls 15.1 to jump over a corresponding one of the radial teeth 11.4 which are shifted or offset along an outer circumference of the end portion 11.1 of the inner housing 11 by 18 degrees in relation to each other. Thus, when the inner housing 11 is rotated, e.g., by 18 degrees, each of the pawls 15.1 will be able to jump over a corresponding one of radial teeth 11.4 of the inner housing 11 only once. As a result, each of the 20 radial teeth 11.4 is arranged at every 18 degrees, and a shift by 120 degrees between each two pawls 15.1 substantially corresponds to 6 and 2/3 (six and two thirds) radial teeth 11.4. The pawls 15.1 are offset or shifted circumferentially with respect to the radial teeth 11.4 such that at every 6 degrees, as a result of the engagement between one of the pawls 15 with a corresponding radial tooth 11.4, a click or cracking sound is generated or produced when the inner housing 11 is rotated in one direction, namely in the direction corresponding to the delivery mode of the set drug dose to the injection site of the patient, or further rotation of the inner housing 11 is blocked when the inner housing 11 is rotated in the opposite direction when accumulating or setting the drug dose to be delivered to the injection site of the patient.

In one embodiment of the present invention, the flexible elements 15, each being provided with a pawl 15.1, may be integrally formed with the bearing sleeve 14 such that the pawls 15.1 of the flexible elements 15 are suitably positioned and oriented within the inner cavity of the bearing sleeve 14 as described above. In another embodiment of the present invention, the flexible elements 15, each being provided with a pawl 15.1, may be secured internally to the bearing sleeve 14 such that the pawls 15.1 of the flexible elements 15 are suitably positioned and oriented within the inner cavity of the bearing sleeve 14 as described above. In a further embodiment of the present invention, the flexible elements 15, each being provided with a pawl 15.1, may each be inserted into a special through-hole or special through-holes provided or formed in the bearing sleeve 14 such that the pawls 15.1 of the flexible elements 15 are suitably positioned and oriented within the inner cavity of the bearing sleeve 14 as described above.

According to one embodiment of the present invention, the bearing sleeve 14 may be provided with at least one flexible element 15 having a pawl 15.1, wherein the flexible element 15 is arranged and oriented so as to engage with the radial teeth 11.4, thereby allowing the flexible element 15 to jump over a corresponding one of the radial teeth 11.4 during each rotation of the inner housing 11 by a predetermined rotation angle corresponding to a single drug dose.

FIG. 16 shows a disc-shaped element 16 fitted onto the seating head 13.2 of the piston rod 13 (see FIGS. 13 and 13a) and capable of engaging with the plunger of the cartridge 4 to allow the required amount of the drug to be discharged from the cartridge 4.

During assembly of the syringe pen 100, the retaining ring 17 is fitted or put onto the inner cylindrical housing 11 from its proximal end until it abuts the retaining flange 11.2. Further, during assembly of the syringe pen 100, flexible elements 15 are mounted on the bearing sleeve 14 such that the free elongated portions 15.4 provided with pawls 15.1 face the inner space of the bearing sleeve 14 at a predetermined angle, and the tips of the pawls 15.1 are arranged in a circle in the inner space of the bearing sleeve 14 and shifted by 120 degrees. Then, the bearing sleeve 14 with the flexible elements 15 mounted thereon is fitted onto the inner housing 11 from its distal end, thereby ensuring the insertion of the elongated arc-shaped protrusions 14.3 into corresponding arc-shaped openings and the engagement of the arc-shaped hooks 14.6 with the retaining ring 17, so that the bearing sleeve 14 substantially completely surrounds the end portion 11.1 of the inner housing and has a required position with respect to the end portion 11.1; the pawls 15.1 of the flexible elements 15 properly engage with corresponding radial teeth 11.4, and the arc-shaped segments are placed into the annular space formed between the arc-shaped sectors 14.1 and the arc-shaped sectors 14.9, wherein the bearing sleeve 14 is no longer able to move axially away from the retaining flange 11.2.

Then, the piston rod 13 is inserted into the inner housing 11 with the bearing sleeve 14 on the side of the proximal end thereof, followed by engaging the screw thread 13.3 of the rod with the screw thread 11.7 of the inner housing 11, thereby ensuring that the distal end of the piston rod 13 is screwed axially into the end portion 11.1 of the inner housing 11 such that a portion of the piston rod 13 extends out of or beyond the distal end of the inner housing 11, as shown, e.g., in FIG. 12a.

Then, the inner housing 11 provided with the bearing sleeve 14 fitted onto the inner housing 11 as described above and the piston rod 13 screwed into the inner housing 11 as described above are installed or inserted into the outer housing 1 from the side of the proximal end thereof such that the extending portion of the piston rod 13 passes through the central opening 1.5 in the partition 1.4 and partially extends beyond the partition 1.4 into the first compartment of the outer housing 1. In addition, when the inner housing 11 is inserted into the outer housing 1 as described above, the arc-shaped portions 14.5 of the bearing sleeve 14 enter the arc-shaped cutouts 1.6 of the partition 1.4 to provide the engagement between the arc-shaped hooks 14.4 and the partition 1.4 of the outer housing 1 from the side of the first compartment thereof, so that the bearing sleeve 14 is fixedly fastened to the outer housing 1, thereby preventing the bearing sleeve 14 from being rotated relative to the outer housing 1 and to the inner housing 11 and further preventing the bearing sleeve from being axially moved relative to the inner housing 11 and to the outer housing 1 so as to ultimately allow the necessary position of the bearing sleeve 14 to be fixed relative to the end portion 11.1 of the inner housing 11.

It is to be noted that as a result of this step of assembling the syringe pen 100, the bearing sleeve 14 is coaxially disposed between the inner housing 11 and the outer housing 1, and the inner housing 11 is prevented from moving axially with respect to the outer housing 1. Furthermore, the insertion of the piston rod 13 into the central opening 1.5 formed in the partition 1.4 of the outer housing 1 prevents or blocks the piston rod 13 from being rotated or pivoted.

During further assembly of the syringe pen 100, the coupling sleeve 7 is fitted or put onto the drum 5 by putting the screw rib 7.4 of the sleeve into screw engagement with the helical groove 5.4 of the drum until the proximal end of the coupling sleeve 7 abuts the annular ledge 5.5 of the drum, thereby disengageably latching the limiting protrusions 5.3 on the limiting cutouts 7.2 and ensuring the screwing of the drum 5 by the distal end thereof into the connecting sleeve 7 from the side of the proximal end thereof. It is to be noted that as a result of fitting the connecting sleeve 7 onto the drum 5, the connecting sleeve 7 is arranged coaxially with the drum 5, and the dose indicator 7.5 indicates the zero dose mark that can be seen through the viewing window 7.3.

Further, during assembly of the syringe pen 100, the drum 5 with the connecting sleeve 7 fitted thereon is inserted by the distal end thereof into the outer housing 1, so that the drum 5 is arranged coaxially with the outer housing 1, and the connecting sleeve 7 is arranged concentrically between the outer housing 1 and the drum 5, wherein the proximal end of the outer housing 1 abuts the annular ledge 5.5 of the drum, so that the head 5.1 of the drum is located outside the outer housing 1 and substantially adjacent to the proximal end of the outer housing 1. Further, when inserting the drum 5 with the connecting sleeve 7 fitted thereon as described above into the outer housing 1 from the side of the distal end of the outer housing 1, the distal end of the connecting sleeve 7 substantially abuts the stop protrusion 1.9 of the outer housing, and the fixing ribs 7.1 of the sleeve frictionally engage with the fixing grooves 1.8 of the outer housing, thereby ensuring the fixation of the connecting sleeve 7 with respect to the outer housing 1 in a predetermined orientation where the viewing window 7.3 is aligned with the viewing window 1.1, so that the dose indicator 7.5 and the drug dose mark indicated by the dose indicator 7.5 are visible through the viewing window 1.1 of the outer housing. Thus, the fixing grooves 1.8 and the fixing ribs 7.1, both being engaged with each other, in combination with the annular stop protrusion 1.9 and the annular ledge 5.5, both having corresponding ends of the connecting sleeve 7 abutted against them, prevent the connecting sleeve 7 from being rotated and axially moved with respect to the outer housing 1 and to the drum 5. It should also be noted that the limiting protrusions 5.3 latched on the limiting cutouts 7.2 prevent the drum 5 from being unintentionally rotated and, accordingly, the drug dose from being unintentionally accumulated or set, wherein in order to set a required drug dose the limiting projections 5.3 have to be released from the engagement with the limiting cutouts 7.2.

Further, during assembly of the syringe pen 100, the annular element 12 is screwed onto the threaded screw 10 such that the annular element 12 is arranged coaxially with the threaded screw 10 and abuts the toothed head 10.1 of the screw, wherein it corresponds to an initial position of the annular element 12 on the toothed screw 10. Further, during assembly of the syringe pen 100, the toothed screw 10 with the annular element 12 screwed onto the screw 10 as described above is rotatably installed in the cylindrical cavity 8.3, so that the mounting heads 10.3 enter corresponding mounting holes 8.4, and the radial spike 12.1 of the annular element enters the guide slot 8.5 of the connecting tube. Thus, the rotation of the screw 10 in the cylindrical cavity 8.3 allows axial rectilinear movement of the annular element 12 along the length of the toothed screw 10 from the toothed screw head 10.1 to the partition 8.7, wherein the position of the annular element 12 on the threaded screw 10 substantially corresponds to the drug dose set by the user. Further, during assembly of the syringe pen 100, the ratchet wheel 9 is fitted or put onto the connecting tube 8 such that the former is arranged coaxially with the connecting tube 8, and the pawls 8.2 engage with corresponding teeth of the toothed rim 9.2.

Then, during assembly of the syringe pen 100, the connecting tube 8 provided with the ratchet wheel 9 fitted onto the connecting tube 8 as described above and with the screw 10 provided with an annular element 12 screwed thereon and installed in the cylindrical cavity 8.3 as described above is installed or inserted into the drum 5 from the side of the proximal end thereof, thereby introducing guide protrusions 8.11 of the connecting tube 8 into threaded engagement with the helical groove 11.6 of the inner housing 11, thereby allowing the threaded portion 11.5 of the inner housing 11 to be screwed into the connecting tube 8 from the side of the distal end thereof substantially until the distal end of the connecting tube 8 abuts the retaining ring 17. As a result of this assembly operation, the connecting tube 8 is concentrically disposed between the inner housing 11 and the drum 5. When the drum 5 is rotated in the dose-setting direction, the connecting tube 8 rotates or pivots so as to rotatably move in an axial direction relative to the outer housing 1 and the inner housing 11, and the inner housing 11 is not pivoted or rotated since the rotation of the inner housing 11 is blocked due to the engagement between one of the pawls 15 with corresponding one of the radial teeth 11.4. When the drum 5 is rotated in the drug dose delivery direction, the entire torque or at least a part thereof is transferred from the connecting tube 8 to the inner housing 11 due to the threaded connection therebetween, resulting in rotation of the inner housing 11 along with the connecting tube 8. In particular, when inserting the connecting tube 8 by the distal end thereof into the drum 5, the shoulder 8.1 of the proximal end of the connecting tube 8 together with the ratchet wheel 9 are completely enclosed in the head 5.1 of the drum, wherein the portion of the ratchet wheel 9 not provided on its outer surface with rims 9.1, 9.2 of inclined axial teeth is arranged in the annular cavity of the head and abuts with its end face against the ends of the ratchet teeth 5.7 at the proximal end of the main portion 5.2 of the drum. Further, when inserting the connecting tube 8 by the distal end thereof into the drum 5, the teeth of the toothed screw head 10.1 engage with the ratchet teeth 5.7 of the drum, allowing the toothed screw 10 to rotate about its axis, wherein the connection formed between the guide slot 8.5 of the connecting tube and the radial spike 12.1 of the annular element introduced into the guide slot 8.5 prevents the annular element 12 from rotating around the threaded screw 10 when transmitting the torque from the drum 5 to the screw 10.

Thus, the rotation of the drum 5 in the drug dose-setting direction, in particular in the clockwise direction, allows the pawls 5.6 of the drum to engage with the teeth of the toothed rim 9.1 and the teeth of the toothed rim 9.2 to engage with the pawls 8.2 of the connecting tube 8, so that the rotational movement of the drum 5 is converted into the rotation of the connecting tube 8 with respect to the inner housing 11 away from its retaining flange 11.2. Meanwhile, the rotation of the drum 5 in the drug dose delivery direction, in particular in the counterclockwise direction, allows the pawls 5.6 of the drum to engage with the teeth of the toothed rim 9.1 and the teeth of the toothed rim 9.2 to engage with the pawls 8.2 of the connecting tube, so that the torque from the drum 5 is transmitted to the connecting tube 8 and further transmitted from the connecting tube 8 to the inner housing 11, resulting in co-rotation of the drum 5, the connecting tube 8 and the inner housing 11 in the same direction, i.e. in the counterclockwise direction.

Further, during assembly of the syringe pen 100, a spring (not shown) is installed or inserted into the seat 8.10 of the connecting tube from the proximal end of the drum 5, so that one end of the spring abuts a bottom of the seat 8.10. Then, the press-button 6 is inserted into the proximal end of the connecting tube 8, so that the main portion 6.3 of the button and the axial pin 6.1 of the button enter the seat 8.10, and the head 6.2 of the button is arranged beyond the head 5.1 of the drum, wherein the spring (not shown) inserted into the seat 8.10 is seated or fitted onto the axial pin 6.1 of the button, so that the other end of the spring abuts against a corresponding one of the horizontal ledges of the axial pin 6.1 of the button, thereby allowing the press-button 6 to be spring-loaded and, accordingly, the press-button 6 to be returned to its initial position after releasing the button 6.

Further, during assembly of the syringe pen 100, a disc-shaped element 16 is fitted or put onto the piston rod head 13.2 formed at the distal end of the piston rod 13.

Further, during assembly of the syringe pen 100, the cartridge 4 is inserted into the holder 2 from the side of the proximal end thereof such that the cartridge head 4.1 is received in the inner cavity of the holder head 2.4.

Further, during assembly of the syringe pen 100, the holder 2 having the cartridge 4 inserted therein is inserted by the first portion thereof into the first compartment of the outer housing 1, so that the longitudinal ribs 2.8 of the holder enter into frictional engagement with corresponding longitudinal grooves 1.3 of the outer housing, and the ends of the sectors 2.10 of the holder at least partially abut the partition 1.4 of the outer housing, and the latching protrusions 2.7 of the holder are latched by the fixing slots 1.2 of the outer housing, and the blocking protrusions 1.7 of the outer housing are inserted into corresponding longitudinal slots 2.9 of the holder until abutting, thereby allowing the first portion of the holder 2 to be tightly fitted in the first compartment of the outer housing 1 until the distal end of the outer housing 1 abuts the flange 2.1 and providing fixation of the holder 2 relative to the outer housing 1 in a predetermined orientation where corresponding smaller viewing window 2.2 and larger viewing window 2.3 are aligned axially with the viewing window 1.1. Thus, when inserting the holder 2 into the outer housing 1, the above fixing means of the holder 2 interact with the above fixing means of the outer housing 1, thereby preventing the holder 2 from being rotated and axially moved relative to the outer housing 1. In addition, when inserting the holder 2 into the outer housing 1, the disc-shaped element 16 engages the plunger of the cartridge 4 to allow a required amount of drug to be discharged from the cartridge 4.

As a final stage of assembly of the syringe pen 100, the cap 3 can be fitted or put onto the holder 2 from the distal end thereof, so that the retaining protrusions 2.5 comes into frictional engagement with the annular recess 3.2, ensuring a tight fit of the cap 3 on the second portion of the holder 2 until the proximal end of the cap 3 abuts the annular flange 2.1. If required, e.g., to check the amount of the drug, to replace or fit a needle sleeve (not shown) onto the cartridge head 4.1 and/or to allow the user to inject the drug remained in the cartridge 4, the cap 3 may be removed from the holder 2 by applying a corresponding force thereto in a direction generally opposite to the direction of fitting the cap 3 onto the holder 2, wherein the applied force needs to allow the annular recess 3.2 of the cap to escape from frictional engagement with the retaining protrusions 2.5 of the holder.

During the initial use of the fully assembled syringe pen 100, the user needs to remove the cap 3 from the holder 2 and to insert or slide the needle sleeve (not shown) onto the cartridge head 4.1.

In order to establish or set a required drug dose, the user needs to grasp the head 5 with user fingers, in particular, by grasping the head if present, and to apply a rotational force thereto in the clockwise direction, the rotational force being sufficient to release the limiting protrusions 5.3 of the drum from engagement with the limiting cutouts 7.2 of the connecting sleeve, thereby causing rotation of the entire drum 5 in the clockwise direction. Meanwhile, as the drum 5 rotates, the drum 5 will gradually extend from the outer housing 1, so that the head 5.1 equipped with the push-button 6 will move away from the proximal end of the outer housing 1. Further, as the drum 5 rotates clockwise, the drum 5 will transmit, via the connection formed between the ratchet teeth 5.7 and the head teeth 10.1 engaged with the ratchet teeth 5.7, a rotation torque from the drum 5 to the screw 10, so that the threaded screw 10 will rotate about its axis in the same direction, allowing axial movement of the annular element 12 along the length of the threaded screw 10 from the screw head 10.1 to the partition 8.7. Meanwhile, when user stops the rotation of the drum 5, the position of the annular element 12 on the threaded screw 10 will substantially correspond to the drug dose set or accumulated by the user. In addition, as the drum 5 rotates clockwise, the user can see one of the scale marks indicated by the indicator 7.5 of the connecting sleeve in the viewing window 1.1, wherein said mark will correspond substantially to the number of units of the drug dose set or accumulated by the user. The drum 5 extending from the outer housing 1 during rotation thereof in the dose-setting direction will substantially pull the connecting tube 8 with it in the direction of extension (provided by means of the above-described toothed connections between the drum 5, the ratchet wheel 9 and the connecting tube 8) to allow the connecting tube 8 to be rotated and axially moved with respect to the inner housing 11 away from the retaining flange 11.2 thereof due to the above-described threaded connection formed between the connecting tube 8 and the inner housing 11.

In order to deliver a required drug dose to an administration or injection site of a patient, the user of the syringe pen 100 applies a generally axial pressure force to the outer surface of the button cap 6.2 for a period of time, resulting in pressing the button 6 and, accordingly, to compression of the spring (not shown), the spring being used to have the button 6 spring-loaded into the seat 8.10. Pressing the button 6 allows at least part of the force applied by the user to be transmitted to the connecting tube 8, which in turn, in particular due to the above-described toothed connections between the drum 5, the ratchet wheel 9 and the connecting tube 8, causes the connecting tube 8 to rotate along with the drum 5 in the counterclockwise direction, i.e. in the direction of delivery of the set drug dose. Due to the above-described threaded connection between the connecting tube 8 and the inner housing 11, the connecting tube 8 transmits the torque to the inner housing 11, causing rotation of the inner housing 11 in the counterclockwise direction. The rotation of the inner housing 11 about its axis in turn provides, due to the above-described threaded connection between the inner housing 11 and the piston rod 13 and due the above-described means for blocking rotation of the piston rod 13, the axial movement of the piston rod 13 provided at the distal end thereof with a disc-shaped element 16 and the discharge of the drug from the cartridge 4 due to the pushing force applied by the disc-shaped element 16 to the plunger (not shown) of the cartridge 4 from the proximal end of the cartridge 4. In other words, the inner housing 11 in the syringe pen 100 serves as an actuating mechanism and is substantially a tubular actuating member converting rotation of the drum 5 into an axial movement of the piston rod 13 by virtue of the above-described functional connections formed between the drum 5, the inner housing 11 and the piston rod 13. Thus, the push-button 6 allows creation of an actuating force which is subsequently transmitted, by means of the drum 5 functionally connected to the connecting tube 8, to the connecting tube 8, then from the connecting tube 8 functionally connected to the inner housing 11 to the inner housing 11, and then from the inner housing 11 functionally connected to the piston rod 13 to the piston rod 13 in order to provide axial movement thereof away from the proximal end of the inner housing 11, wherein the magnitude of axial movement of the rod 13 substantially corresponds to the drug dose set or accumulated by the user. As a result, the axial movement of the piston rod 13 upon rotation of the drum 5 in the direction of delivery of the predetermined drug dose results in the advance of the plunger 4 towards the distal end of the cartridge 4 by means of the disc-shaped element 16 and allows the drug discharged from the cartridge 4 to be supplied to the site of injection or administration of the drug to the patient, wherein the magnitude of movement of the piston rod 13 and, accordingly, the magnitude of the advancement of the plunger of the cartridge 4 by the piston rod 13 depend solely on the level of the drug dose in dose units as previously set by means of the drum 5, as described above.

It is to be noted that during the axial movement of the piston rod 13 caused by the rotation of the drum 5 in the direction of delivery of the set drug dose, the inner housing 11 rotates or pivots and, therefore, the pawls 15.1 of the flexible elements engage with corresponding radial teeth 11.4 of the inner housing depending on the amount of the drug dose set or accumulated by the user as to be delivered to the injection site of the patient, providing at least one jump of at least one of the pawls 15.1 over corresponding radial teeth 11.4, wherein the jump of the pawls 15.1 over corresponding radial teeth 11.4 occurs sequentially or alternately, thereby allowing generation of a sound in the form of clicks or other sharp sounds audible to the user. Thus, if the inner housing 11 is rotated, e.g., by 24 degrees, one of the pawls 15.1 jumps over corresponding one of the radial teeth at each 6 degrees of rotation, while the jump of the pawls 15.1 occurs sequentially or alternately: firstly, when the inner housing 11 is rotated by the first 6 degrees, one of the pawls 15.1 jumps over and generates the first click, then, when the inner housing 11 is rotated by the next 6 degrees, another one of the pawls 15.1 jumps over and generates the second click, then, when the inner housing 11 is rotated by the next 6 degrees, the remaining pawl 15.1 jumps over and generates the third click, and when the inner housing 11 is rotated by the remaining 6 degrees, the first of the pawls 15.1 having the first click initially generated by its jump jumps over again and generates the fourth click, and therefore one of the pawls 15.1 will make two jumps and each of the other two pawls 15.1 will make one jump, thereby generating four consecutive clicks. The sound generated by the tip of one of the pawls 15.1 passing through the tip of a particular one of the radial teeth 11.4 as the inner housing 11 rotates in the delivery mode of a set drug dose to the injection site of the patient, is generally represented by sound signals in the form of clicks and cracks generated by the syringe pen 100, thereby allowing the user, in particular the visually impaired user or the user with distractible attention, to control, by means of hearing, the course of the delivery of the set drug dose to the injection site on the patient's body. Thus, the radial teeth 11.4 of the inner housing, in combination with the flexible elements 15, each flexible element 15 being provided with a pawl 15.1 designed to engage with a corresponding one of the radial teeth 11.4 of the inner housing, substantially form a sound-generating mechanism or an auditory feedback mechanism indicating to the user, by audible click or crack signals, the beginning, continuation and/or completion of the delivery of the set drug dose to the injection site.

It should further be noted that, in the present invention, at least one of the following aforementioned structural parts of the disclosed pen 100 can be formed by a cylindrical part, a cylindrical element, a cylindrical housing, a tubular housing, a tube, a tubular element, a tubular part, a sleeve, or the like: the outer housing 1, the inner housing 11, the drum 5, the connecting tube 8, and the bearing sleeve.

The above pen 100 can be used to administer or inject a mammal (e.g., a human or animal) with a drug selected from the group consisting of insulins, insulin analogs, hormones, heparins, antihistamines, and their derivatives and analogs.

## Claims

1. A drug delivery device, comprising:
a housing,
a drug reservoir installed in the housing,
a rod movably installed in the housing so as to enable the drug to be discharged from the reservoir,
an actuating mechanism capable of generating an actuating force,
a dose-setting mechanism installed at least partially in the housing, wherein the dose-setting mechanism is capable of setting a drug dose and operatively coupled to the actuating mechanism,
**characterized in that** the drug delivery device further comprises
a tubular member provided with radial teeth, wherein the tubular member is operatively coupled in the housing to the dose-setting mechanism and to the rod such that the actuating force is transmitted to the rod in order to move the latter depending on the set drug dose, and
a cylindrical member provided with at least one flexible element having a pawl, wherein the cylindrical member is locked in the housing such that the pawl is capable of jumping over at least one of the radial teeth during rotation of the tubular member.

2. The device according to claim 1, wherein the cylindrical member is provided with three flexible elements, each comprising a pawl, and wherein the cylindrical member is locked such that the pawls are arranged around the tubular member and offset with respect to each other at a predetermined offset angle, thereby allowing at least one of the pawls to alternatively jump over corresponding one of the radial teeth during rotation of the tubular member.

3. The device according to claim 2, wherein each rotation of the tubular member by a predetermined angle corresponding to a unit of the drug dose allows only one of the pawls to jump over corresponding one of the radial teeth of the tubular member.

4. The device according to claim 3, wherein the predetermined rotation angle of the tubular member is 6 degrees, and the radial teeth of the tubular member are offset by 18 degrees, and the pawls of the flexible elements are offset by 120 degrees.

5. The use of the drug delivery device according to any one of claims 1-4 for injecting a drug selected from a group consisting of insulins, insulin analogs, hormones, heparins, antihistamines, and their derivatives and analogs.
